# EUROPÄISCHE PATENTSCHRIFT

(11) **EP 1 100 453 B1**
(45) Veröffentlichungstag und Bekanntmachung des Hinweises auf die Patenterteilung: **05.01.2005**
(21) Anmeldenummer: 99936589.3
(22) Anmeldetag: 22.07.1999
(51) Int. Cl.: A61K 7/48

(54) **KOSMETISCHE FORMULIERUNG ENTHALTEND ECTOINDERIVATE, ENZYME, VITAMINE UND/ODER VITAMIN-DERIVATE**
COSMETIC FORMULATION CONTAINING ECTOINE DERIVATIVES, ENZYMES, VITAMINS AND/OR VITAMIN DERIVATIVES
FORMULATION COSMETIQUE CONTENANT DES DERIVES D'ECTOINE, DES ENZYMES, DES VITAMINES ET/OU DES DERIVES VITAMINIQUES

(30) Priorität: 01.08.1998 DE 19834818
(43) Veröffentlichungstag der Anmeldung: 23.05.2001
(73) Patentinhaber: MERCK PATENT GmbH, 64293 Darmstadt (DE)
(72) Erfinder: BÜNGER, Joachim, D-64823 Gro -Umstadt (DE); DRILLER, Hans-Jürgen, D-64823 Gro -Umstadt (DE)
(86) Internationale Anmeldenummer: PCT/EP1999/005238
(87) Internationale Veröffentlichungsnummer: WO 2000/007559

(56) Entgegenhaltungen:
- EP-A- 0 647 469
- DE-A- 2 614 723
- DE-A- 2 746 650
- DE-A- 4 342 560
- GB-A- 2 114 886
- DATABASE WPI Week 199732 Derwent Publications Ltd., London, GB; AN 1997-347460 XP002121426 "Stabilisation of enzymes - comprises adding ectoin to enzymes e.g. oxidoreductase" & JP 09 143167 A (DAINIPPON PHARM), 3. Juni 1997 (1997-06-03)
- J. BUENGER: "Neue Wirkstoffklasse schützt und pflegt die Haut" PARFUEMERIE UND KOSMETIK., Bd. 79, Nr. 11, November 1998 (1998-11), Seiten 32-35, XP000852629 HUETHIG, HEIDELBERG., DE ISSN: 0031-1952

## Beschreibung

Die Erfindung betrifft kosmetische Formulierungen enthaltend
a) eine oder mehrere Verbindungen ausgewählt aus den Verbindungen der Formeln la und Ib 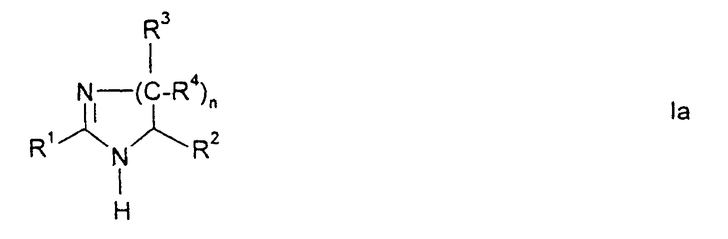 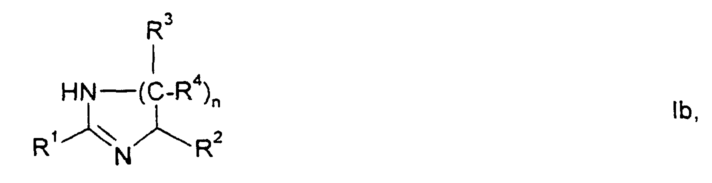 den physiologisch verträglichen Salzen der Verbindungen der Formeln la und Ib, und den stereoisomeren Formen der Verbindungen der Formeln la und Ib, wobei
   - R¹: H oder Alkyl,
   - R²: H, COO-H, COO-Alkyl oder CO-NH-R⁵,
   - R³ und R⁴: jeweils unabhängig voneinander H oder OH,
   - n: 1, 2 oder 3,
   - Alkyl: einen Alkylrest mit 1 bis 4 Kohlenstoffatomen, und
   - R⁵: H, Alkyl, einen Aminosäurerest, Dipeptidrest oder Tripeptidrest
   bedeuten,
b) eine oder mehrere Enzyme
   und
c) Hilfs- und Trägerstoffe,
dadurch gekennzeichnet, daß sie zusätzlich ein oder mehrere Vitamine und/oder Vitamin-Derivate ausgewählt sind aus Vitamin A, Vitamin-A-Propionat, Vitamin-A-Palmitat, Vitamin-A-Acetat, Retinol, Vitamin B, Thiaminchloridhydrochlorid, Riboflavin, Nicotinsäureamid, Vitamin C, Vitamin D, Ergocalciferol, Vitamin E, DL-α-Tocopherol, Tocopherol-E-Acetat, Tocopherolhydrogensuccinat, Vitamin K₁, Esculin, Thiamin, Nicotinsäure, Pyridoxin, Pyridoxal, Pyridoxamin, Panthothensäure, Biotin, Folsäure und Cobalamin enthält.

Enzyme, Vitamine und Vitamin-Derivate besitzen häufig eine geringe Stabilität und sind in vitro einer Reihe denaturierender bzw. destabilisierender Bedingungen ausgesetzt. Beispielsweise sind derartige Substanzen empfindlich gegenüber Veränderungen des sie umgebenden Mediums und gegenüber Temperaturschwankungen. Während Vitamine und Vitamin-Derivate zwar in der Regel als Pulver stabil sind, trifft dies für wäßrige Formulierungen prinzipiell nicht zu. Die Lagerung von Enzymen, Vitaminen und Vitamin-Derivaten über einen längeren Zeitraum führt daher zu einer Abnahme ihrer Aktivität. Besonders schwierig ist die Anwendung derartiger Substanzen in Kosmetika, da diese topisch applizierten Produkte großen Temperatur- und Feuchtigkeitsschwankungen auf der Haut ausgesetzt sind und nach dem gleichmäßigen Verteilen auf der Haut in der Regel schnell austrocknen. Dadurch kann eine Denaturierung bzw. Destabilisierung der Enzyme, Vitamine und Vitamin-Derivate auftreten, bevor diese aus dem Depot auf der Hautoberfläche an ihren Wirkort in tiefere Schichten der Epidermis gelangen.

Sollen dennoch Enzyme, Vitamine und Vitamin-Derivate als Bestandteile von Kosmetika verwendet werden, müssen diese in hohen Konzentrationen eingesetzt und durch geeignete Transport- bzw. Carrier-Systeme möglichst schnell zum Wirkort transportiert werden.

Das Problem der Abnahme der Aktivität von Enzymen, Vitaminen und Vitamin-Derivaten tritt insbesondere auch in Formulierungen auf, in denen diese Substanzen über einen längeren Zeitraum eine möglichst hohe Aktivität besitzen müssen. Dies ist beispielsweise bei Formulierungen mit einer gleichmäßigen Freisetzung der Enzyme, Vitamine und Vitamin-Derivate über einen längeren Zeitraum, was auch als "Depot-Effekt" bezeichnet wird, der Fall.

Aus den obengenannten Gründen ist es notwendig, Enzyme, Vitamine und Vitamin-Derivate in kosmetischen Formulierungen zu stabilisieren und vor Denaturierung zu schützen.

Es bestand daher die Aufgabe, kosmetische Formulierungen enthaltend Substanzen ausgewählt aus Vitaminen und Vitamin-Derivaten zur Verfügung zu stellen, die sich dadurch auszeichnen, daß derartige in der Formulierung enthaltene Substanzen eine möglichst langandauerd hohe Aktivität besitzen und die kosmetischen Formulierungen darüber hinaus in vorteilhafter Weise zur Pflege und Prophylaxe einer trockenen und/oder schuppigen Haut geeignet sind.

Überraschend wurde nun gefunden, daß diese Aufgabe durch die Bereitstellung von kosmetischen Formulierungen enthaltend
a) eine oder mehrere Verbindungen ausgewählt aus den Verbindungen der Formeln Ia und Ib 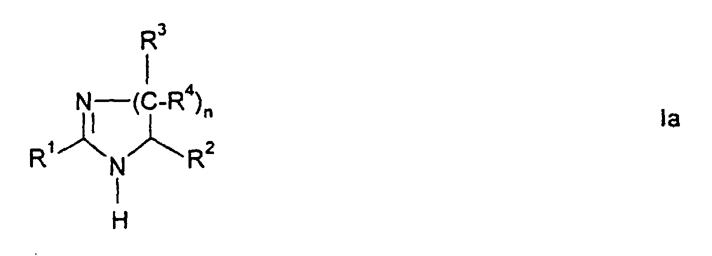 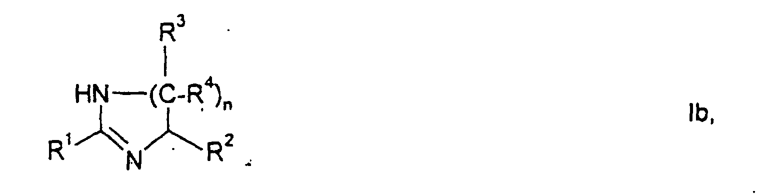 den physiologisch verträglichen Salzen der Verbindungen der Formeln Ia und Ib, und den stereoisomeren Formen der Verbindungen der Formeln la und Ib, wobei
   - R¹: H oder Alkyl,
   - R²: H, COOH, COO-Alkyl oder CO-NH-R⁵,
   - R³ und R⁴: jeweils unabhängig voneinander H oder OH,
   - n: 1, 2 oder 3,
   - Alkyl: einen Alkylrest mit 1 bis 4 Kohlenstoffatomen, und
   - R⁵: H, Alkyl, einen Aminosäurerest, Dipeptidrest oder Tripeptidrest
   bedeuten
b) eine oder mehrere Enzyme
   und
c) Hilfs- und Trägerstoffe,
dadurch gekennzeichnet, daß sie zusätzlich ein oder mehrere Vitamine und/oder Vitamin-Derivate ausgewählt sind aus Vitamin A, Vitamin-A-Propionat, Vitamin-A-Palmitat, Vitamin-A-Acetat, Retinol, Vitamin B, Thiaminchloridhydrochlorid, Riboflavin, Nicotinsäureamid, Vitamin C, Vitamin D, Ergocalciferol, Vitamin E, DL-α-Tocopherol, Tocopherol-E-Acetat, Tocopherolhydrogensuccinat, Vitamin K₁, Esculin, Thiamin, Nicotinsäure, Pyridoxin, Pyridoxal, Pyridoxamin, Panthothensäure, Biotin, Folsäure und Cobalamin enthält.
gelöst wird.

Im Rahmen der vorliegenden Erfindung werden alle vor- und nachstehenden Verbindungen ausgewählt aus den Verbindungen der Formeln Ia und Ib, den physiologisch verträglichen Salzen der Verbindungen der Formeln Ia und Ib, und den stereoisomeren Formen der Verbindungen der Formeln Ia und Ib als "Ectoin oder Ectoin-Derivate" bezeichnet.

Die Erfindung betrifft weiterhin
- die Verwendung von einer oder mehreren Substanzen ausgewählt aus ausgewählt aus den Verbindungen der Formeln Ia und Ib, den physiologisch verträglichen Salzen der Verbindungen der Formeln Ia und Ib, und den stereoisomeren Formen der Verbindungen der Formeln Ia und Ib und mit ein oder mehreren Enzymen und Hilfs- und Trägerstoffen zur Herstellung einer kosmetischen Formulierung, dadurch gekennzeichnet, dass die Formulierung zusätzlich ein oder mehrere Vitamine und/oder Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid, Riboflavin, Nicotinsäureamid, Vitamin C, Vitamin D, Ergocalciferol, Vitamin E, DL-α-Tocopherol, Tocopherol-E-Acetat, Tocopherolhydrogensuccinat, Vitamin K₁, Esculin, Thiamin, Nicotinsäure, Pyridoxin, Pyridoxal, Pyridoxamin, Panthothensäure, Biotin, Folsäure und Cobalamin enthält.
- ein Verfahren zur Herstellung einer kosmetischen Formulierung, dadurch gekennzeichnet, daß man eine oder mehrere Verbindungen ausgewählt aus den Verbindungen der Formeln Ia und Ib, den physiologisch verträglichen Salzen der Verbindungen der Formeln la und Ib, und den stereoisomeren Formen der Verbindungen der Formeln Ia und Ib und eine oder mehrere Enzyme mit Hilfs- und/oder Trägerstoffen in eine geeignete kosmetische Formulierungsform bringt, die zusätzlich ein oder mehrere Vitamine und/oder Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid, Riboflavin, Nicotinsäureamid, Vitamin C, Vitamin D, Ergocalciferol, Vitamin E, DL-α-Tocopherol, Tocopherol-E-Acetat, Tocopherolhydrogensuccinat, Vitamin K₁, Esculin, Thiamin, Nicotinsäure, Pyridoxin, Pyridoxal, Pyridoxamin, Panthothensäure, Biotin, Folsäure und Cobalamin enthält.
- die Verwendung einer oder mehrerer Verbindungen ausgewählt aus den Verbindungen der Formeln Ia und Ib, den physiologisch verträglichen Salzen der Verbindungen der Formeln Ia und Ib, und den stereoisomeren Formen der Verbindungen der Formeln la und Ib zum Schutz und/oder zur Stabilisierung von einer oder mehreren in einer kosmetischen Formulierung enthaltenen Substanzen ausgewählt aus Vitaminen und Vitamin-Derivaten.

Die in den erfindungsgemäßen kosmetischen Formulierungen enthaltenen Ectoine oder Ectoin-Derivate schützen die Substanzen ausgewählt aus Vitaminen und Vitamin-Derivaten in der Formulierung gegen Stressfaktoren, wie Schwankungen des Wassergehalts und der Tempera-tur nach topischer Applikation auf die Haut. Zudem schützt Ectoin oder seine Derivate die genannten Substanzen gegen hohe Salz- oder Ionenkonzentrationen in der erfindungsgemäßen kosmetischen Formulierung. Durch die stabilisierende Wirkung des Ectoins oder der Ectoin-Derivate, werden die in der kosmetischen Formulierung enthaltenen Substanzen ausgewählt aus Enzymen, Vitaminen und Vitamin-Derivaten nach dem Auftragen auf die Hautoberfläche langsamer destabilisiert bzw. denaturiert, so daß ein Depot-Effekt mit hoher Aktivität der Substanzen ausgewählt aus Enzymen, Vitaminen und Vitamin-Derivaten möglich ist. Die Substanzen ausgewählt aus Vitaminen und Vitamin-Derivaten werden durch Ectoin oder seine Derivate aber auch vor der topischen Applikation in der Formulierung geschützt. Dies wirkt sich in vorteilhafter Weise in einer Erhöhung der Lagerstabilität einer Formulierung, die derartige Substanzen enthält, aus.

Der Schutz der Substanzen ausgewählt aus Vitaminen und Vitamin-Derivaten in der kosmetischen Formulierung durch Ectoin oder seine Derivate wirkt sich in unterschiedlicher Weise vorteilhaft auf die menschliche Haut aus. Beispielsweise werden dadurch die antioxidativen Wirkungen, die gegen das Altern der Haut gerichteten Wirkungen ("antiage-Wirkungen"), die gegen die Faltenbildung gerichteten Wirkungen, die hautglättenden Wirkungen und die keratolytischen Wirkungen dieser Substanzen auf die menschliche Haut unterstützt bzw. geschützt, d.h. dem vorzeitigen Verlust oder der vorzeitigen Verminderung dieser Wirkungen durch den Verlust oder die Abnahme der Aktivität der Substanzen ausgewählt aus Vitaminen und Vitamin-Derivaten wird entgegengewirkt.

Offenbart wird weiterhin die Verwendung von
a) einer oder mehreren Verbindungen ausgewählt aus den Verbindungen der Formeln la und Ib 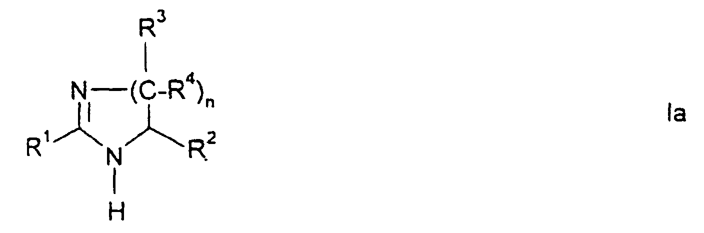 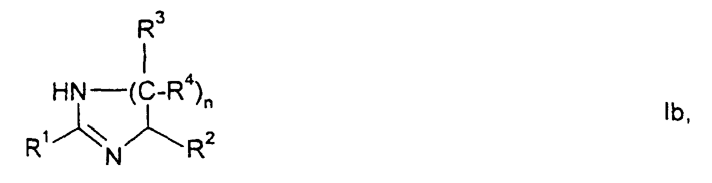 den physiologisch verträglichen Salzen der Verbindungen der Formeln la und Ib. und den stereoisomeren Formen der Verbindungen der Formeln Ia und Ib, wobei
   - R¹: H oder Alkyl,
   - R²: H, COOH, COO-Alkyl oder CO-NH-R⁵,
   - R³ und R⁴: jeweils unabhängig voneinander H oder OH,
   - n: 1, 2 oder 3,
   - Alkyl: einen Alkylrest mit 1 bis 4 Kohlenstoffatomen, und
   - R⁵: H, Alkyl, einen Aminosäurerest, Dipeptidrest oder Tripeptidrest
   bedeuten
   und
b) einer oder mehreren Enzyme
c) Hilfs- und Trägerstoffen,
zur Herstellung einer kosmetischen Formulierung.
- zur Pflege und/oder Prophylaxe einer trockenen und/oder schuppigen Haut oder Kopfhaut, insbesondere zur Erhöhung und/oder Stabilisierung des Feuchtigkeitsgehalts der Haut,
- zum Schutz der menschlichen Haut gegen Stressfaktoren, insbesondere gegen Trockenheit durch hohe Temperaturen oder sehr niedrige Temperaturen bei geringer Luftfeuchtigkeit und/oder gegen hohe Salzkonzentration auf der Haut,
- zum Schutz von Zellen, Proteinen, und/oder Biomembranen der menschlichen Haut,
- zum Schutz der Mikroflora der menschlichen Haut und/oder
- zur Stabilisierung der Hautbarriere.

Die gesunde menschliche Haut wird an ihrer Oberfläche, dem Stratum corneum, von einer großen Anzahl kommensalisch lebender Mikroorganismen kolonisiert. Aus der großen Vielfalt dieser Mikroorganismen leben nur wenige ständig auf der Haut und bilden so die residente Hautflora. Die Hauptvertreter der residenten Flora auf der menschlichen Haut sind Staphylococcen, Micrococcen, coryneforme Bakterien und Pityrosporen. Diese leben in kleinen Kolonien auf der Oberfläche des Stratum corneum und in der äußeren Epidermis. Eine zweite Gruppe von Mikroorganismen, die sich vorübergehend von außen, insbesondere auf exponierten Hautbereichen, ansiedelt, wird als transiente Flora bezeichnet und kann sich auf der gesunden Haut, deren Mikromilieu stark durch die residente Mikroflora bestimmt wird, nicht dauerhaft ansiedeln. in unterschiedlichen Körperregionen variiert die Zusammensetzung der Hautflora in Abhängigkeit vom Mikromilieu der Haut. Die Dichte der Mikroorganismen paßt sich dem jeweiligen Hautmilieu an, so daß die Ökologie dieser Körperregionen nicht durch eine übermäßige Besiedlung durch Mikroorganismen aus dem Gleichgewicht gebracht wird. Im Vergleich zum Normalzustand der Haut nimmt die Anzahl der Mikroorganismen bei trockener Haut ab, während die Anzahl der Mikroorganismen bei feuchter Haut, z.B. durch entzündliche Veränderungen bei einem Ekzem, bis um das 1000-fache zunimmt.

Die Haut ist als Grenzschicht und Oberfläche des menschlichen Körpers einer Vielzahl externer Streßfaktoren ausgesetzt. Die Human-Haut ist ein Organ, das mit verschiedenartig spezialisierten Zelltypen - den Keratinozyten, Melanozyten, Langerhans-Zellen, Merkel-Zellen und eingelagerten Sinneszellen - den Körper vor äußeren Einflüssen schützt. Hierbei ist zwischen äußeren physikalischen, chemischen und biologischen Einflüssen auf die menschliche Haut zu unterscheiden. Zu den äußeren physikalischen Einflüssen sind thermische und mechanische Einflüsse sowie die Einwirkung von Strahlen zu zählen. Unter den äußeren chemischen Einflüssen sind insbesondere die Einwirkung von Toxinen und Allergenen zu verstehen. Die äußeren biologischen Einflüsse umfassen die Einwirkung fremder Organismen und deren Stoffwechselprodukte.

Die Oberfläche der menschlichen Haut wird von einem Fettfilm bedeckt, der, je nach den gegebenen Verhältnissen, als eine Öl-in-Wasser- oder eine Wasser-in-Öl-Emulsion anzusehen ist und zahlreiche Wirkstoffe, wie z.B. Enzyme und Vitamine, enthält. Dieser Fettfilm, der aus den von Talgdrüsen und Keratinozyten abgegebenen Lipiden gebildet wurde, bewahrt die Feuchtigkeit der Haut und schützt den Körper als Hautbarriere vor ungünstigen Umweltfaktoren. Dieses empfindliche Gleichgewicht der Hautbarriere wird durch externe oder interne Faktoren gestört.

Die Mikroorganismen der menschlichen Haut sind verschiedenen Streßfaktoren ausgesetzt. Beispielsweise können sie durch Austrocknung oder durch hohe Salzkonzentrationen auf der Hautoberfläche, z.B. nach dem Schwitzen, geschädigt werden, was eine Schädigung der Hautbarriere zur Folge haben kann. Einige dieser Mikroorganismen - Staphylococcen, Micrococcen, Corynebakterien und Brevibakterien - besitzen jedoch üblicherweise die Fähigkeit, Kompatible Solute zu bilden, um sich gegen Austrocknung oder hohe Salzkonzentration zu schützen und tragen somit zur Ausbildung einer intakten Hautbarriere bei. Die Kompatiblen Solute, die auch als Streßschutzstoffe bezeichnet werden, sind niedermolekulare Substanzen im Cytoplasma.

Bisher wurde hauptsächlich der Versuch unternommen, den Erhalt der Hautfeuchtigkeit durch hydrophile Substanzen, die selbst Wasser binden, zu bewirken (E.A. Galinski, Experientia 49 (1993) 487-496). Diese hydrophilen Substanzen binden jedoch Wassermoleküle des Hydratationswassers ebenso wie freie Wassermoleküle. Dadurch kommt es zwar zu einer Bindung von Wassermolekülen, nicht jedoch zu einem Schutz der Hydrathüllen von Zellen, Proteinen und Zellmembranen.

Ectoinhaltige kosmetische Formulierungen schützen Zellen, Proteine, Enzyme, Vitamine, DNA, Zell- und Biomembranen der Haut vor den Schäden durch Austrocknung und Wasserentzug. Durch die Hydratationswirkung des Ectoins wird das Wassergleichgewicht des Stratum corneums sowie die Hautbarriere stabilisiert. Ectoin beugt einer trockenen und schuppigen Haut vor.

Ectoinhaltige kosmetische Formulierungen schützen zudem die für eine intakte Hautbarriere wichtige Mikroflora der Haut gegen Streß durch Austrocknung und hohe Ionenkonzentration nach dem Schwitzen. Die Stabilisierung der residenten Hautflora durch Ectoin oder seine Derivate ist eine wichtige Voraussetzung für das Gleichgewicht des Mikromilieus der Haut und die Ausbildung einer intakten Hautbarriere.

Bei Ectoin und den Ectoin-Derivaten handelt es sich um niedermolekulare, cyclische Aminosäurederivate, die aus verschiedenen halophilen Mikroorganismen gewonnen werden können. Sowohl Ectoin als auch Hydroxyectoin besitzen den Vorteil, daß sie nicht mit dem Zellstoffwechsel reagieren.

In der DE 43 42 560 wird die Verwendung von Ectoin und Ectoin-Derivaten als Feuchtigkeitsspender in Kosmetikprodukten beschrieben.

Die Verbindungen ausgewählt aus den Verbindungen der Formeln Ia und Ib, den physiologisch verträglichen Salzen der Verbindungen der Formeln la und Ib und den stereoisomeren Formen der Verbindungen der Formeln la und Ib können in den kosmetischen Zubereitungen als optische Isomere, Diastereomere, Racemate, Zwitterionen, Kationen oder als Gemisch derselben vorliegen. Unter den Verbindungen ausgewählt aus den Verbindungen der Formeln la und Ib, den physiologisch verträglichen Salzen der Verbindungen der Formeln la und Ib und den stereoisomeren Formen der Verbindungen der Formeln Ia und Ib, sind diejenigen Verbindungen bevorzugt, worin R¹ H oder CH₃, R² H oder COOH, R³ und R⁴ jeweils unabhängig voneinander H oder OH und n 2 bedeuten. Unter den Verbindungen ausgewählt aus den Verbindungen der Formeln la und Ib, den physiologisch verträglichen Salzen der Verbindungen der Formeln la und Ib und den stereoisomeren Formen der Verbindungen der Formeln la und Ib sind die Verbindungen (S)-1,4,5,6-Tetrahydro-2-methyl-4-pyrimidincarbonsäure (Ectoin) und (S,S)-1,4,5,6-Tetrahydro-5-hydroxy-2-methyl-4-pyrimidincarbonsäure (Hydroxyectoin) insbesondere bevorzugt.

Unter dem Begriff "Aminosäure" werden die stereoisomeren Formen, z.B. D- und L-Formen, folgender Verbindungen verstanden: Alanin, β-Alanin, Arginin, Asparagin, Asparaginsäure, Cystein, Glutamin, Glutaminsäure, Glycin, Histidin, Isoleucin, Leucin, Lysin, Methionin, Phenylalanin, Serin, Threonin, Tryptophan, Tyrosin, Valin, γ-Aminobutyrat, Nε-Acetyllysin, Nδ-Acetylornithin, Nγ-Acetyldiaminobutyrat und Nα-Acetyldiaminobutyrat. L-Aminosäuren sind bevorzugt.

Aminosäurereste leiten sich von den entsprechenden Aminosäuren ab.

Die Reste folgender Aminosäuren sind bevorzugt: Alanin, β-Alanin, Asparagin, Asparaginsäure, Glutamin, Glutaminsäure, Glycin, Serin, Threonin, Valin, γ-Aminobutyrat, Nε-Acetyllysin, Nδ-Acetylornithin, Nγ-Acetyldiaminobutyrat und Nα-Acetyldiaminobutyrat.

Die Di- und Tripeptidreste sind ihrer chemischen Natur nach Säureamide und zerfallen bei der Hydrolyse in 2 oder 3 Aminosäuren. Die Aminosäuren in den Di- und Tripeptidresten sind durch Amidbindungen miteinander verbunden. Bevorzugte Di- und Tripetidreste sind aus den bevorzugten Aminosäuren aufgebaut.

Die Alkylgruppen umfassen die Methylgruppe CH₃, die Ethylgruppe C₂H₅, die Propylgruppen CH₂CH₂CH₃ und CH(CH₃)₂ sowie die Butylgruppen CH₂CH₂CH₂CH₃, H₃CCHCH₂CH₃, CH₂CH(CH₃)₂ und C(CH₃)₃. Die bevorzugte Alkylgruppe ist die Methylgruppe.

Bevorzugte physiologisch verträgliche Salze der Verbindungen der Formeln la und Ib sind beispielsweise Alkali-, Erdalkali- oder Ammoniumsalze, wie Na-, K-, Mg- oder Ca-Salze, sowie Salze abgeleitet von den organischen Basen Triethylamin oder Tris-(2-hydroxy-ethyl)-amin. Weitere bevorzugte physiologisch verträgliche Salze der Verbindungen der Formeln la und Ib ergeben sich durch Umsetzung mit anorganischen Säuren wie Salzsäure, Schwefelsäure und Phosphorsäure oder mit organischen Carbon- oder Sulfonsäuren wie Essigsäure, Citronensäure, Benzoesäure, Maleinsäure, Fumarsäure, Weinsäure und p-Toluolsulfonsäure.

Verbindungen der Formeln la und Ib, in denen basische und saure Gruppen wie Carboxyl- oder Aminogruppen in gleicher Zahl vorliegen, bilden innere Salze.

Die Herstellung der Verbindungen der Formel Ia und Ib ist in der Literatur beschrieben (DE 43 42 560). (S)-1,4,5,6-Tetrahydro-2-methyl-4-pyrimidincarbonsäure oder (S,S=-1,4,5,6-Tetrahydro-5-hydroxy-2-methyl-4-pyrimidincarbonsäure können auch mikrobiologisch gewonnen werden (Severin et al., J. Gen. Microb. 138 (1992) 1629-1638).

Bevorzugt sind Enzyme ausgewählt aus Superoxiddismutase, Protease, Lipase, Pepsin, Trypsin, Chymotrypsin, Elastase, Diastase, Katalase, Dehydrogenase, Urease, Lysozym, Neuraminidase, Peroxidase, Glutathionperoxidase, Phosphatase, Dismutase, Papain, Bromelain, Alcalase, Aminopeptidase K, Aminopeptidase M, Carboxypeptidase A, Carboxypeptidase B, Carboxypeptidase Y, Clostripain, Collagenase, Ficin, Leucin-Amidopeptidase, Lytisches Enzym, Pronase E und Proteinase K in den erfindungsgemäßen kosmetischen Formulierungen enthalten, insbesondere bevorzugt Superoxiddismutase, Protease, Lipase und Dismutase.

Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherof, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂) in den erfindungsgemäßen kosmetischen Formulierungen enthalten, insbesondere bevorzugt Vitamin-A-Palmitat, Vitamin C, DL-α-Tocopherol, Tocopherol-E-Acetat, Nicotinsäure, Pantothensäure und Biotin.

Alle in den erfindungsgemäßen Formulierungen enthaltenen Enzyme, Vitamine und Vitamin-Derivate lassen sich nach bekannten Methoden synthetisieren oder sind käuflich erwerbbar.

Die Herstellung der kosmetischen Formulierung erfolgt, indem eine oder mehrere Verbindungen ausgewählt aus den Verbindungen der Formeln la und Ib, den physiologisch verträglichen Salzen der Verbindungen der Formeln Ia und Ib und den stereoisomeren Formen der Verbindungen der Formeln Ia und Ib und eine oder mehrere Substanzen ausgewählt aus Enzymen, Vitaminen und Vitamin-Derivaten gegebenenfalls mit Hilfsund/oder Trägerstoffen in eine geeignete Formulierungsform gebracht werden. Die Hilfs- und Trägerstoffe stammen aus der Gruppe der Trägermittel, Konservierungsstoffe und anderer üblicher Hilfsstoffe.

Die kosmetischen Formulierungen auf der Grundlage einer oder mehrerer Verbindungen ausgewählt aus den Verbindungen der Formeln la und Ib, den physiologisch verträglichen Salzen der Verbindungen der Formeln la und Ib und den stereoisomeren Formen der Verbindungen der Formeln la und Ib und einer oder mehreren Substanzen ausgewählt aus Enzymen, Vitaminen und Vitamin-Derivaten werden äußerlich angewendet.

Als Anwendungsform seien z.B. genannt: Lösungen, Suspensionen, Emulsionen, Pasten, Salben, Gele, Cremes, Lotionen, Puder, Seifen, tensidhaltige Reinigungspräparate, Öle und Sprays. Zusätzlich zu einer oder mehreren Verbindungen ausgewählt aus den Verbindungen der Formeln Ia und Ib, den physiologisch verträglichen Salzen der Verbindungen der Formeln la und Ib und den stereoisomeren Formen der Verbindungen der Formeln la und Ib und einer oder mehreren Substanzen ausgewählt aus Enzymen, Vitaminen und Vitamin-Derivaten werden der Formulierung beliebige übliche Trägerstoffe, Hilfsstoffe und gegebenenfalls weitere Wirkstoffe zugesetzt.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer.

Salben, Pasten, Cremes und Gele können neben einer oder mehreren Verbindungen ausgewählt aus den Verbindungen der Formeln la und Ib, den physiologisch verträglichen Salzen der Verbindungen der Formeln la und Ib und den stereoisomeren Formen der Verbindungen der Formeln la und Ib und einer oder mehreren Substanzen ausgewählt aus Enzymen, Vitaminen und Vitamin-Derivaten die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können neben einer oder mehreren Verbindungen ausgewählt aus den Verbindungen der Formeln la und Ib, den physiologisch verträglichen Salzen der Verbindungen der Formeln la und Ib und den stereoisomeren Formen der Verbindungen der Formeln Ia und Ib und einer oder mehreren Substanzen ausgewählt aus Enzymen, Vitaminen und Vitamin-Derivaten die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können neben einer oder mehreren Verbindungen ausgewählt aus den Verbindungen der Formeln la und Ib, den physiologisch verträglichen Salzen der Verbindungen der Formeln la und Ib und den stereoisomeren Formen der Verbindungen der Formeln Ia und Ib und einer oder mehreren Substanzen ausgewählt aus Enzymen, Vitaminen und Vitamin-Derivaten die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnußöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können neben einer oder mehreren Verbindungen ausgewählt aus den Verbindungen der Formeln la und Ib, den physiologisch verträglichen Salzen der Verbindungen der Formeln Ia und Ib und den stereoisomeren Formen der Verbindungen der Formeln la und Ib und einer oder mehreren Substanzen ausgewählt aus Enzymen, Vitaminen und Vitamin-Derivaten die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können neben einer oder mehreren Verbindungen ausgewählt aus den Verbindungen der Formeln Ia und Ib, den physiologisch verträglichen Salzen der Verbindungen der Formeln la und Ib und den stereoisomeren Formen der Verbindungen der Formeln la und Ib und einer oder mehreren Substanzen ausgewählt aus Enzymen, Vitaminen und Vitamin-Derivaten die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können neben einer oder mehreren Verbindungen ausgewählt aus den Verbindungen der Formeln la und Ib, den physiologisch verträglichen Salzen der Verbindungen der Formeln la und Ib und den stereoisomeren Formen der Verbindungen der Formeln la und Ib und einer oder mehreren Substanzen ausgewählt aus Enzymen, Vitaminen und Vitamin-Derivaten die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können neben einer oder mehrerer Verbindungen ausgewählt aus den Verbindungen der Formeln Ia und Ib, den physiologisch verträglichen Salzen der Verbindungen der Formeln la und Ib und den stereoisomeren Formen der Verbindungen der Formeln Ia und Ib und einer oder mehreren Substanzen ausgewählt aus Enzymen, Vitaminen und Vitamin-Derivaten die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typisch kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Der Anteil der Verbindungen ausgewählt aus den Verbindungen der Formeln la und Ib, den physiologisch verträglichen Salzen der Verbindungen der Formeln la und Ib und den stereoisomeren Formen der Verbindungen der Formeln Ia und Ib in der erfindungsgemäßen kosmetischen Formulierung beträgt vorzugsweise von 0,0001 bis 50 Gew.%, besonders bevorzugt von 0,001 bis 10 Gew.% bezogen auf die gesamte kosmetische Formulierung.

Der Anteil der Enzyme in der erfindungsgemäßen kosmetischen Formulierung beträgt vorzugsweise von 0,0001 bis 10 Gew.%, besonders bevorzugt von 0,001 bis 5 Gew.% bezogen auf die gesamte kosmetische Formulierung.

Der Anteil der Vitamine und/oder Vitamin-Derivate in der erfindungsgemäßen kosmetischen Formulierung beträgt vorzugsweise von 0,0001 bis 50 Gew.%, besonders bevorzugt von 0,001 bis 20 Gew.% bezogen auf die gesamte kosmetische Formulierung.

Der Anteil der Verbindungen ausgewählt aus den Verbindungen der Formeln Ia und Ib, den physiologisch verträglichen Salzen der Verbindungen der Formeln Ia und Ib und den stereoisomeren Formen der Verbindungen der Formeln Ia und Ib zusammen mit den Substanzen ausgewählt aus Enzymen, Vitaminen und Vitamin-Derivaten in der erfindungsgemäßen kosmetischen Formulierung beträgt vorzugsweise von 0,0001 bis 50 Gew.%, besonders bevorzugt von 0,001 bis 20 Gew.% bezogen auf die gesamte kosmetische Formulierung.

Das Gewichtsverhältnis der Verbindungen ausgewählt aus den Verbindungen der Formeln Ia und Ib, den physiologisch verträglichen Salzen der Verbindungen der Formeln Ia und Ib und den stereoisomeren Formen der Verbindungen der Formeln la und Ib zu den Enzymen in der erfindungsgemäßen kosmetischen Formulierung beträgt vorzugsweise von 1 : 1 bis 1000 : 1, besonders bevorzugt von 1 : 1 bis 100 : 1.

Das Gewichtsverhältnis der Verbindungen ausgewählt aus den Verbindungen der Formeln Ia und Ib, den physiologisch verträglichen Salzen der Verbindungen der Formeln la und Ib und den stereoisomeren Formen der Verbindungen der Formeln la und Ib zu den Vitaminen und/oder Vitamin-Derivaten der erfindungsgemäßen kosmetischen Formulierung beträgt vorzugsweise von 1 : 100 bis 1000 : 1, besonders bevorzugt von 1 : 10 bis 100 : 1.

Die Bestimmung der Enzym-Aktivität kann sowohl in vivo als auch in vitro nach bekannten Methoden erfolgen. In vitro-Bestimmungen der Enzymaktivität können beispielsweise nach Einwirkung von Streßfaktoren wie z.B. Hitze oder Trockenheit mit validierten Enzymtests, die für viele Enzyme käuflich erwerbbar sind, wie z.B. das "Elastase Kit", erfolgen. In vivo-Bestimmungen der Aktivität von Hautenzymen, wie z.B. Protease oder Dismutase, erfolgen beispielsweise derart, daß ein bestimmtes Hautareal zunächst mit Ectoin vorbehandelt wird und anschließend einem Trockenstreß ausgesetzt wird. Nach Entfernen der Enzyme auf der Haut, z.B. durch Tape-stripping, wird eine Enzymbestimmung nach bekannten Methoden durchgeführt. Nach dem schichtweise erfolgenden Abziehen der Hautzellen läßt sich die Enzymaktivität der einzelnen Zellschichten bestimmen.

Im Rahmen der vorliegenden Erfindung wurde untersucht, ob die für einen Zellschutz wichtigen Enzyme Katalase, Glutathionperoxidase, Superoxiddismutase und Elastase durch die Behandlung mit Ectoin gegenüber einem Temperaturstress geschützt werden können.

Bei aeroben Stoffwechselprozessen entsteht unter der Einwirkung von Oxidasen in den Peroxisomen (Zellorganellen) Wasserstoffperoxid, das von der gleichzeitig anwesenden Katalase sofort zersetzt wird. Nach proteolytischer Aktivierung durch Trypsin spaltet Elastase hydrolytisch die Peptid-Ketten des Fibrins, Hämoglobins, Albumins, Caseins und anderer Proteine, insbesondere aber des Elastins. Elastase findet sich z.B. auch in bestimmten Leukozyten, wo sie an der Phagozytose und Abwehr von Mikroorganismen beteiligt ist. Um die Zerstörung körpereigenen Bindegewebes durch die Elastase zu verhindern, muß ihre Aktivität sorgfältig kontrolliert werden, was im Blutkreislauf durch Inhibitoren α1-Antitrypsin und andere, z.B. α2-Makroglobin, geschieht.

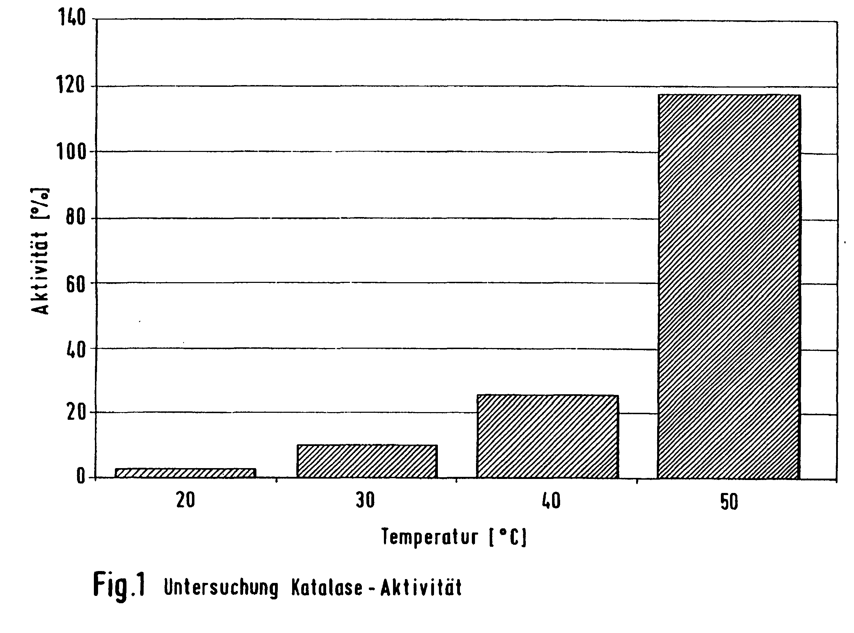

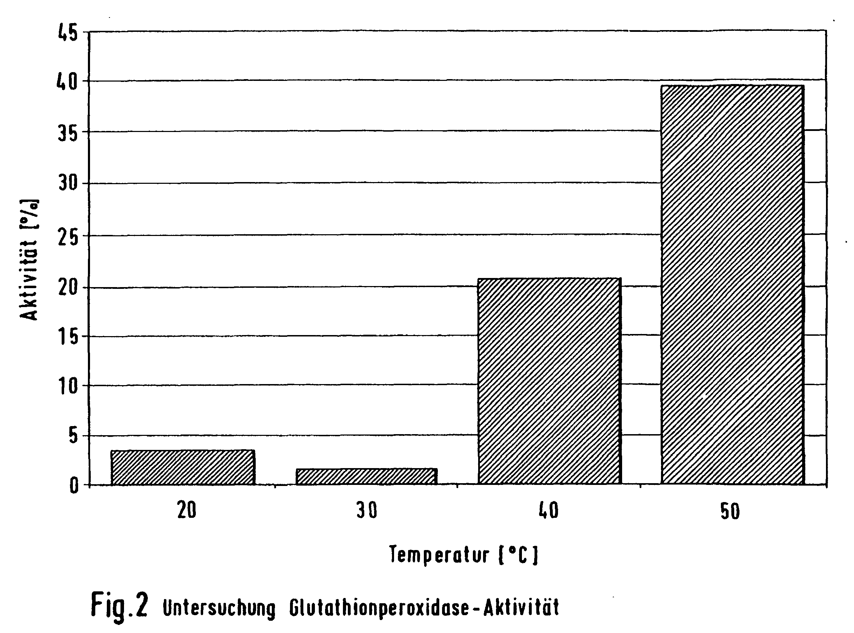

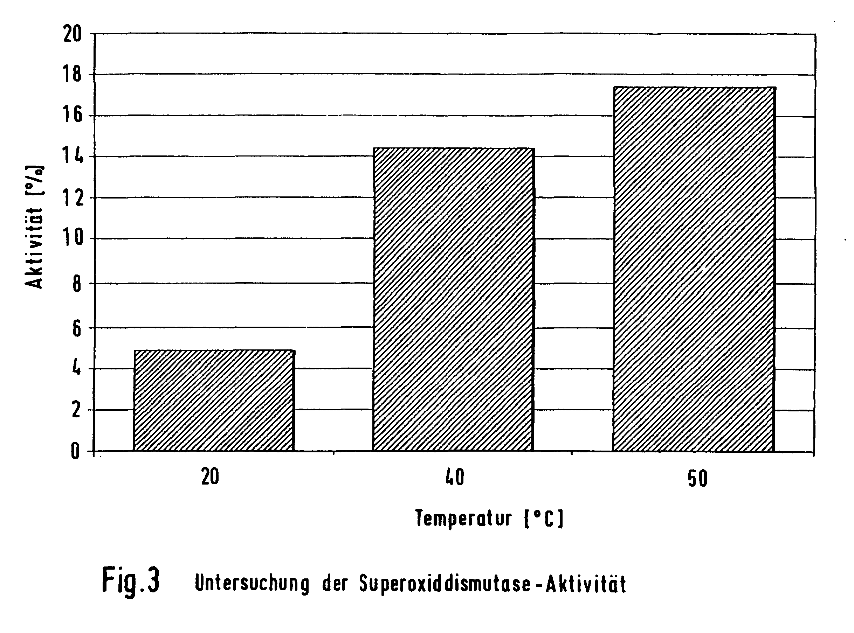

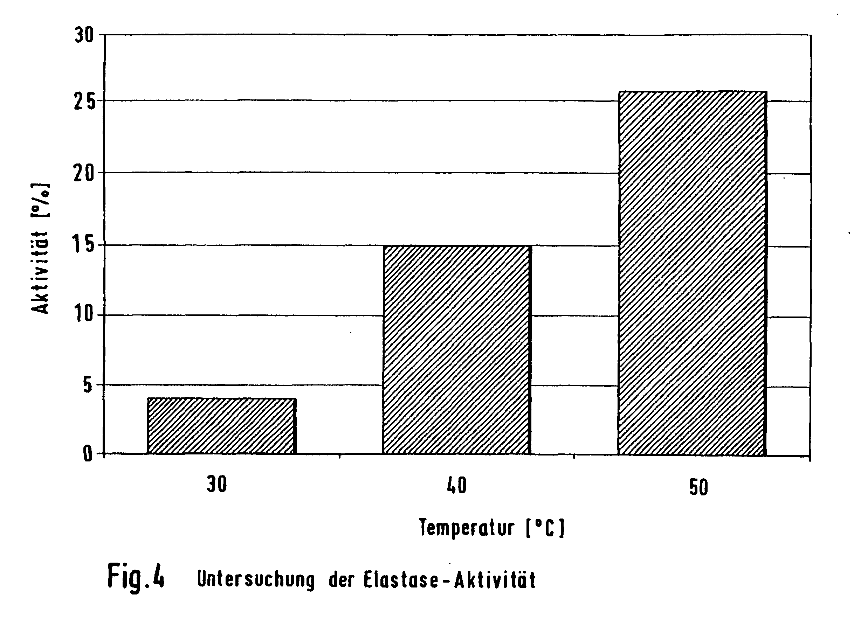

Es wurde gefunden, daß Katalase, Glutathionperoxidase, Superoxiddismutase und Elastase gegenüber einem Temperaturstress stabiler sind, wenn sie mit Ectoin behandelt werden. Die Stabilisierung dieser mit Ectoin behandelten Enzyme ist in Abb. 1 bis Abb. 4 dargestellt, insbesondere zeigt Abb. 1 die Stabilisierung von Katalase, Abb. 2 die Stabilisierung von Glutathionperoxidase, Abb. 3 die Stabilisierung von Superoxiddimutase und Abb. 4 die Stabilisierung von Elastase. In den Abbildungen ist jeweils der Unterschied der Enzymaktivität des mit Ectoin behandelten Enzyms im Vergleich zum entsprechenden nicht mit Ectoin behandelten Enzym dargestellt, wobei die Aktivität des unbehandelten Enzyms 100 % gleichgesetzt wurde. Beispielsweise ist nach Inkubation bei einer Temperatur von 40 °C die Aktivität von mit Ectoin behandelter Katalase um ca. 25 % höher als von nicht mit Ectoin behandelter Katalase (s. Abb. 1). Nach Inkubation bei einer Temperatur von 50 °C ist die Aktivität von mit Ectoin behandelter Katalase dagegen um fast 120 % höher als von nicht mit Ectoin behandelter Katalase, d.h. mit Ectoin behandelte Katalase ist nach Inkubation bei dieser Temperatur mehr als doppelt so aktiv wie nicht mit Ectoin behandelte Katalase (s. Abb. 1). Tendenziell ist die stabilisierende Wirkung des Ectoins auf die Enzym-Aktivität für alle untersuchten Enzyme für höhere Inkubationstemperaturen stärker ausgeprägt. Die größten Effekte werden jeweils für Inkubationstemperaturen von 40 °C und vor allem von 50 °C festgestellt, insbesondere für die Enzyme Katalase und Glutathionperoxidase.

Zur Feststellung der stabilisierenden Wirkung von Ectoin oder seinen Derivaten auf Vitamine und Vitamin-Derivate kann deren chemische und physikalische Stabilität durch Standard-Tests nachgewiesen werden.

Alle Verbindungen oder Komponenten, die in den kosmetischen Formulierungen verwendet werden können, sind entweder.bekannt und käuflich erwerbbar oder können nach bekannten Methoden synthetisiert werden.

Die folgenden Beispiele dienen zur Verdeutlichung der Erfindung und sind keinesfalls als Limitierung aufzufassen. Alle %-Angaben sind Gewichtsprozent.

### Beispiel 1

Aus folgenden Komponenten wird ein erfindungsgemäßes Hautpflegegel (O/W) enthaltend Ectoin und Vitamin C hergestellt:

| | | | Gew.-% |
|---|---|---|---|
| A | Mandelöl | (2) | 8.0 |
| | Eutanol G | (3) | 2.0 |
| | Luvitol EHO | (4) | 6.0 |
| | Oxynex K flüssig (Art.-Nr. 108324) | (1) | 0.05 |
| | | | |
| B | Vitamin C (Art.-Nr. 500074) | (1) | 0.5 |
| | Karion F flüssig (Art.-Nr. 102993) | (1) | 4.0 |
| | Konservierungsmittel | | q.s. |
| | Wasser, demineralisiert | | ad 100 |
| | | | |
| C | Sepigel 305 | (5) | 3.0 |
| | | | |
| D | Ectoin | (1) | 1.0 |

Als Konservierungsmittel können
0.05 % Propyl-4-hydroxybenzoat (Art.-Nr. 107427) oder
0.15 % Methyl-4-hydroxybenzoat (Art.-Nr. 106757)
verwendet werden.

### Herstellung:

Die vereinigte Phase B wird unter Rühren langsam in die Phase C eingetragen. Danach wird die vorgelöste Phase A zugesetzt. Es wird gerührt bis die Phasen homogen gemischt sind. Anschließend wird Phase D zugegeben und bis zur Homogenität gerührt.

### Bezugsquellen:

(1) Merck KGaA, Darmstadt
(2) Gustav Heess, Stuttgart
(3) Henkel KGaA, Düsseldorf
(4) BASF AG, Ludwigshafen
(5) Seppic, Frankreich

### Beispiel 2

Aus folgenden Komponenten wird ein erfindungsgemäßes Hautpflegegel (O/W) enthaltend Ectoin und Protease hergestellt:

| | | | Gew.-% |
|---|---|---|---|
| A | Mandelöl | (2) | 8.0 |
| | Eutanol G | (3) | 2.0 |
| | Luvitol EHO | (4) | 6.0 |
| | Oxynex K flüssig (Art.-Nr. 108324) | (1) | 0.05 |
| | | | |
| B | Protease (Pepsin) (Art.-Nr. 107185) | (1) | 0.01 |
| | Karion F flüssig (Art.-Nr. 102993) | (1) | 4.0 |
| | Konservierungsmittel | | q.s. |
| | Wasser, demineralisiert | | ad 100 |
| | | | |
| C | Sepigel 305 | (5) | 3.0 |
| | | | |
| D | Ectoin | (1) | 1.0 |

Als Konservierungsmittel können
0.05 % Propyl-4-hydroxybenzoat (Art.-Nr. 107427) oder
0.15 % Methyl-4-hydroxybenzoat (Art.-Nr. 106757)
verwendet werden.

### Herstellung:

Die vereinigte Phase B wird unter Rühren langsam in die Phase C eingetragen. Danach wird die vorgelöste Phase A zugesetzt. Es wird gerührt bis die Phasen homogen gemischt sind. Anschließend wird Phase D zugegeben und bis zur Homogenität gerührt.

### Bezugsquellen:

(1) Merck KGaA, Darmstadt
(2) Gustav Heess, Stuttgart
(3) Henkel KGaA, Düsseldorf
(4) BASF AG, Ludwigshafen
(5) Seppic, Frankreich

### Beispiel 3

Es wird die enzymstabilisierende Wirkung von Ectoin gegen Temperaturstress in vitro untersucht. Hierzu werden die Enzyme Katalase, Glutathionperoxidase, Superoxiddismutase und Elastase mit Ectoin - und zur Kontrolle ohne Ectoin - 30 Minuten im Wasserbad bei verschiedenen Temperarturen inkubiert und danach ihre Aktivität gemessen. Das Grundmedium für die Inkubation ist eine 0,25 molare Tris-HCl-Pufferlösung mit einem pH-Wert von 6,8. Die Enzymkonzentration beträgt 0,05 mg/ml und die Konzentration an Ectoin beträgt 1 mol/l.

Die Bestimmung der Enzymaktivität erfolgt
- für Katalase nach der Methode von A. Claiborne [A. Claiborne 'Catalase Activity' in: R.A. Greenwald (Ed.), Handbook of Methods for Oxygen Radical Research, CRC Press, Boca Raton, FL. (1986) 283-284],
- für Glutathionperoxidase nach der Methode von L. Flohé und W.A. Günzler [L. Flohé et al., Methods Enzymol. 105 (1989) 114-121],
- für Superoxiddismutase nach der Methode von H.P. Nissen und H.W. Kreysel [H.W. Kreysel et al., 'Superoxide Dismutase in Human Semen, Klin. Wochenschr. 61 (1983) 63-65] und
- für Elastase nach der Methode von L. Hornebeck und W. Hornebeck [W. Hornebeck et al., 'Characterisation of Human Skin Fibroblasts Elastase Activity', J. Invest. Dermatol. 91 (5) (1988) 472-477].

Die Ergebnisse sind für Katalase in Abb. 1, für Glutathionperoxidase in Abb. 2, für Superoxiddimutase in Abb. 3 und für Elastase in Abb. 4 dargestellt. In den Abbildungen ist jeweils der Unterschied der Enzymaktivität des mit Ectoin behandelten Enzyms im Vergleich zum entsprechenden nicht mit Ectoin behandelten Enzym als 'Aktivität [%] für die verschiedenen Inkubationstemperaturen dargestellt, wobei die Aktivität des unbehandelten Enzyms 100 % gleichgesetzt wurde. Für eine Inkubationstemperatur von 50 °C ist die Aktivität von mit Ectoin behandelter Katalase demnach um fast 120 % höher als von nicht mit Ectoin behandelter Katalase, d.h. für diese Inkubationstemperatur ist die Aktivität von mit Ectoin behandelter Katalase ca. 2,2 Mal so hoch wie die Aktivität von nicht mit Ectoin behandelter Katalase (s. Abb. 1).

Die Werte zu Abb. 1 bis 4 sind in Tab. 1 bis 4 aufgeführt.

**Tab. 1**

| Untersuchung der Katalase-Aktivität. | | | | |
|---|---|---|---|---|
| | Temperatur [°C] | | | |
| | 20 | 30 | 40 | 50 |
| Aktivität [%] | 2,5 | 10,4 | 26,1 | 117,9 |

**Tab. 2**

| Untersuchung der Glutathionperoxidase-Aktivität. | | | | |
|---|---|---|---|---|
| | Temperatur [°C] | | | |
| | 20 | 30 | 40 | 50 |
| Aktivität [%] | 3,5 | 1,4 | 20,8 | 39,6 |

**Tab. 3**

| Untersuchung der Superoxiddismutase-Aktivität. | | | |
|---|---|---|---|
| | Temperatur [°C] | | |
| | 20 | 40 | 50 |
| Aktivität [%] | 4,9 | 14,4 | 17,4 |

**Tab. 4**

| Untersuchung der Elastase-Aktivität. | | | |
|---|---|---|---|
| | Temperatur [°C] | | |
| | 30 | 40 | 50 |
| Aktivität [%] | 4,0 | 15,0 | 25,7 |

Es wurde gefunden, daß Katalase, Glutathionperoxidase, Superoxiddismutase und Elastase gegenüber einem Temperaturstress stabiler sind, wenn sie mit Ectoin behandelt werden. Tendenziell ist die stabilisierende Wirkung des Ectoins auf die Enzym-Aktivität für alle untersuchten Enzyme für höhere Inkubationstemperaturen stärker ausgeprägt. Die größten Effekte werden jeweils für Inkubationstemperaturen von 40 °C und 50 °C festgestellt, insbesondere für die Enzyme Katalase und Glutathionperoxidase.

## Patentansprüche

1. Kosmetische Formulierung enthaltend
a) eine oder mehrere Verbindungen ausgewählt aus den Verbindungen der Formeln Ia und Ib 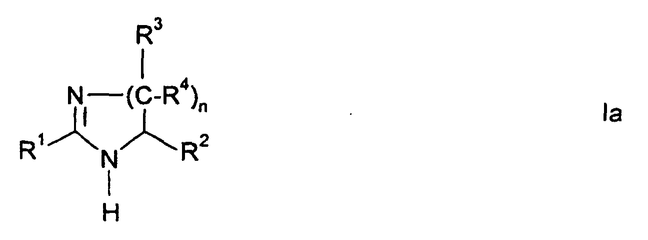 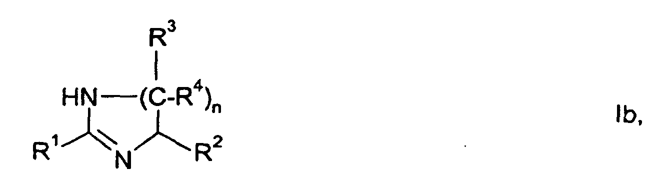 den physiologisch verträglichen Salzen der Verbindungen der Formeln la und Ib, und den stereoisomeren Formen der Verbindungen der Formeln Ia und Ib, wobei
R¹ H oder Alkyl,
R² H, COOH, COO-Alkyl oder CO-NH-R⁵,
R³ und R⁴ jeweils unabhängig voneinander H oder OH,
n 1, 2 oder 3,
Alkyl einen Alkylrest mit 1 bis 4 Kohlenstoffatomen, und
R⁵ H, Alkyl, einen Aminosäurerest, Dipeptidrest oder Tripeptidrest
bedeuten,
b) eine oder mehrere Enzyme
und
c) Hilfs- und Trägerstoffe,
**dadurch gekennzeichnet, daß** sie zusätzlich ein oder mehrere Vitamine und/oder Vitamin-Derivate ausgewählt sind aus Vitamin A, Vitamin-A-Propionat, Vitamin-A-Palmitat, Vitamin-A-Acetat, Retinol, Vitamin B, Thiaminchloridhydrochlorid, Riboflavin, Nicotinsäureamid, Vitamin C, Vitamin D, Ergocalciferol, Vitamin E, DL-α-Tocopherol, Tocopherol-E-Acetat, Tocopherolhydrogensuccinat, Vitamin K₁, Esculin, Thiamin, Nicotinsäure, Pyridoxin, Pyridoxal, Pyridoxamin, Panthothensäure, Biotin, Folsäure und Cobalamin enthält.

2. Kosmetische Formulierung nach Anspruch 1, **dadurch gekennzeichnet, daß** die Verbindungen der Formeln Ia und Ib ausgewählt sind aus den Verbindungen (S)-1,4.5,6-Tetrahydro-2-methyl-4-pyrimidincarbonsäure und (S,S)-1,4,5,6-Tetrahydro-5-hydroxy-2-methyl-4-pyrimidincarbonsäure.

3. Kosmetische Formulierung nach einem der Ansprüche 1 oder 2, **dadurch gekennzeichnet, daß** die Enzyme ausgewählt sind aus Superoxiddismutase, Protease, Lipase, Pepsin, Trypsin, Chymotrypsin, Elastase, Diastase, Katalase, Dehydrogenase, Urease, Lysozym, Neuraminidase, Peroxidase, Glutathionperoxidase, Phosphatase, Dismutase, Papain, Bromelain, Alcalase, Aminopeptidase K, Aminopeptidase M, Carboxypeptidase A, Carboxypeptidase B, Carboxypeptidase Y, Clostripain, Collagenase, Ficin, Leucin-Amidopeptidase, Lytisches Enzym, Pronase E und Proteinase K.

4. Kosmetische Formulierung nach einem der Ansprüche 1 bis 3, **dadurch gekennzeichnet, daß** der Anteil der Verbindungen ausgewählt aus den Verbindungen der Formeln la und Ib, den physiologisch verträglichen Salzen der Verbindungen der Formeln la und Ib, und den stereoisomeren Formen der Verbindungen der Formeln la und Ib von 0,0001 bis 50 Gew.% bezogen auf die gesamte kosmetische Formulierung beträgt und der Anteil der Enzyme von 0,0001 bis 10 Gew.% bezogen auf die gesamte kosmetische Formulierung beträgt.

5. Kosmetische Formulierung nach einem der Ansprüche 1 bis 4, **dadurch gekennzeichnet, daß** der Anteil der Verbindungen ausgewählt aus den Verbindungen der Formeln la und Ib, den physiologisch verträglichen Salzen der Verbindungen der Formeln la und Ib, und den stereoisomeren Formen der Verbindungen der Formeln la und Ib von 0,0001 bis 50 Gew.% bezogen auf die gesamte kosmetische Formulierung beträgt und der Anteil der Vitamine und/oder Vitamin-Derivate von 0,001 bis 20 Gew.% bezogen auf die gesamte kosmetische Formulierung beträgt.

6. Kosmetische Formulierung nach einem der Ansprüche 1 bis 5, **dadurch gekennzeichnet, daß** sie in Form einer Lösung, einer Suspension, einer Emulsion, einer Paste, einer Salbe, eines Gels, einer Creme, einer Lotion, eines Puders, einer Seife, eines tensidhaltigen Reinigungspräparates, eines Öls, eines Lippenstifts, eines Lippenpflegestifts, einer Mascara, eines Eyeliners, von Lidschatten, von Rouge, eines Puder-, Emulsions- oder Wachs-Make ups, eines Sonnenschutz-, Prä-Sun- und After-Sun-Präparats oder eines Sprays vorliegt.

7. Verwendung von
a) einer oder mehreren Verbindungen ausgewählt aus den Verbindungen der Formeln Ia und Ib 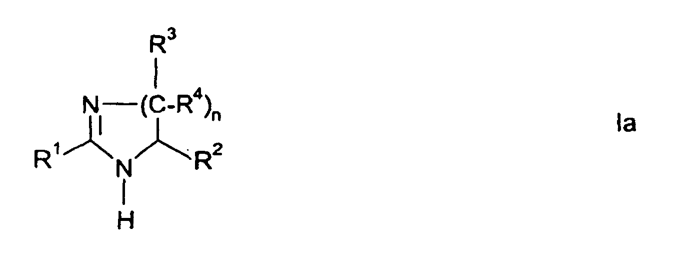 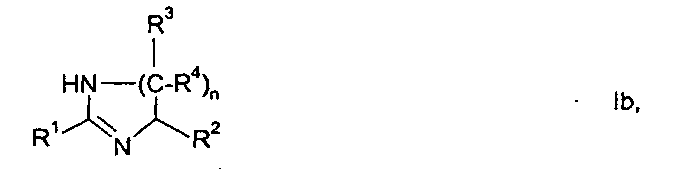 den physiologisch verträglichen Salzen der Verbindungen der Formeln Ia und Ib, und den stereoisomeren Formen der Verbindungen der Formeln Ia und Ib, wobei
R¹ H oder Alkyl,
R² H, COOH, COO-Alkyl oder CO-NH-R⁵,
R³ und R⁴ jeweils unabhängig voneinander H oder OH,
n 1, 2 oder 3,
Alkyl einen Alkylrest mit 1 bis 4 Kohlenstoffatomen, und
R⁵ H, Alkyl, einen Aminosäurerest, Dipeptidrest oder Tripeptidrest
bedeuten,
zum Schutz und/oder zur Stabilisierung von einer oder mehreren in einer kosmetischen Formulierung enthaltenen Substanzen ausgewählt aus Vitaminen und Vitamin-Derivaten.

8. Verwendung von
a) einer oder mehreren Verbindungen ausgewählt aus den Verbindungen der Formeln Ia und Ib 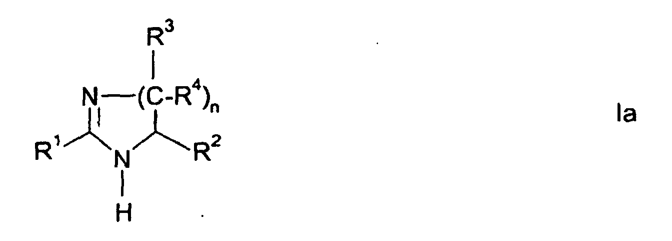 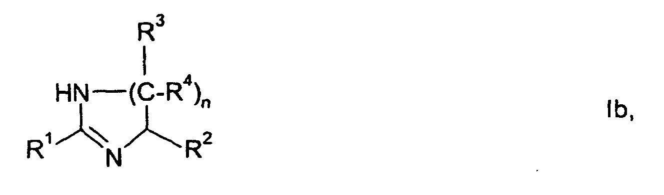 den physiologisch verträglichen Salzen der Verbindungen der Formeln la und Ib, und den stereoisomeren Formen der Verbindungen der Formeln Ia und Ib, wobei
R¹ H oder Alkyl,
R² H, COOH, COO-Alkyl oder CO-NH-R⁵,
R³ und R⁴ jeweils unabhängig voneinander H oder OH,
n 1, 2 oder 3,
Alkyl einen Alkylrest mit 1 bis 4 Kohlenstoffatomen, und
R⁵ H, Alkyl, einen Aminosäurerest, Dipeptidrest oder Tripeptidrest
bedeuten,
b) ein oder mehreren Enzymen und
c) Hilfs- und Trägerstoffen
zur Herstellung einer kosmetischen Formulierung, dadurch gegekennzeichnet, dass die Formulierung zusätzlich ein oder mehrere Vitamine und/oder Vitamin-Derivate ausgewählt aus Vitamin A, Vitamin-A-Propionat, Vitamin-A-Palmitat, Vitamin-A-Acetat, Retinol, Vitamin B, Thiaminchtoridhydrochlorid, Riboflavin, Nicotinsäureamid, Vitamin C, Vitamin D, Ergocalciferol, Vitamin E, DL-α-Tocopherol, Tocopherot-E-Acetat, Tocopherolhydrogensuccinat, Vitamin K₁, Esculin, Thiamin, Nicotinsäure, Pyridoxin, Pyridoxal, Pyridoxamin, Panthothensäure, Biotin, Folsäure und Cobalamin enthält.

9. Verwendung nach Anspruch 8, **dadurch gekennzeichnet, daß** die kosmetische Formulierung in Form einer Lösung, einer Suspension, einer Emulsion, einer Paste, einer Salbe, eines Gels, einer Creme, einer Lotion, eines Puders, einer Seife, eines tensidhaltigen Reinigungspräparates, eines Öls, eines Lippenstifts, eines Lippenpflegestifts, einer Mascara, eines Eyeliners, von Lidschatten, von Rouge, eines Puder-, Emulsions- oder Wachs-Make ups, eines Sonnenschutz-, Prä-Sun- und After-Sun-Präparats oder eines Sprays vorliegt.

10. Verwendung nach einem der Ansprüche 7 bis 9, **dadurch gekennzeichnet, daß** die Verbindungen der Formeln Ia und Ib ausgewählt sind aus den Verbindungen (S)-1,4,5,6-Tetrahydro-2-methyl-4-pyrimidincarbonsäure und (S,S)-1,4,5,6-Tetrahydro-5-hydroxy-2-methyl-4-pyrimidincarbonsäure.

11. Verwendung nach einem der Ansprüche 7 bis 10, **dadurch gekennzeichnet, daß** die Enzyme ausgewählt sind aus Superoxiddismutase, Protease, Lipase, Pepsin, Trypsin, Chymotrypsin, Elastase, Diastase, Katalase, Dehydrogenase, Urease, Lysozym, Neuraminidase, Peroxidase, Glutathionperoxidase, Phosphatase, Dismutase, Papain, Bromelain, Alcalase, Aminopeptidase K, Aminopeptidase M, Carboxypeptidase A, Carboxypeptidase B, Carboxypeptidase Y, Clostripain, Collagenase, Ficin, Leucin-Amidopeptidase, Lytisches Enzym, Pronase E und Proteinase K.

12. Verwendung nach einem der Ansprüche 7, 10 oder 11, **dadurch gekennzeichnet, daß** die Vitamine und/oder Vitamin-Derivate ausgewählt sind aus Vitamin A, Vitamin-A-Propionat, Vitamin-A-Palmitat, Vitamin-A-Acetat, Retinol, Vitamin B, Thiaminchloridhydrochlorid, Riboflavin, Nicotinsäureamid, Vitamin C, Vitamin D, Ergocalciferol, Vitamin E, DL-α-Tocopherol, Tocopherol-E-Acetat, Tocopherolhydrogensuccinat, Vitamin K₁, Esculin, Thiamin, Nicotinsäure, Pyridoxin, Pyridoxal, Pyridoxamin, Panthothensäure, Biotin, Folsäure und Cobalamin.

13. Verfahren zur Herstellung einer kosmetischen Formulierung, **dadurch gekennzeichnet, daß** man
a) eine oder mehrere Verbindungen ausgewählt aus den Verbindungen der Formeln Ia und Ib 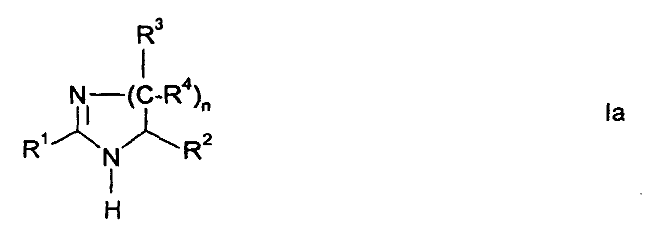 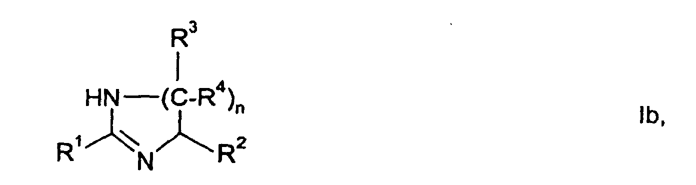 den physiologisch verträglichen Salzen der Verbindungen der Formeln Ia und Ib, und den stereoisomeren Formen der Verbindungen der Formeln Ia und Ib, wobei
R¹ H oder Alkyl,
R² H, COOH, COO-Alkyl oder CO-NH-R⁵,
R³ und R⁴ jeweils unabhängig voneinander H oder OH,
n 1, 2 oder 3,
Alkyl einen Alkylrest mit 1 bis 4 Kohlenstoffatomen, und
R⁵ H, Alkyl, einen Aminosäurerest, Dipeptidrest oder Tripeptidrest
bedeuten
und
b) eine oder mehrere Enzyme
mit Hilfs- und/oder Trägerstoffen in eine geeignete kosmetische Formulierungsform bringt, die zusätzlich ein oder mehrere Vitamine und/oder Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid, Riboflavin, Nicotinsäureamid, Vitamin C, Vitamin D, Ergocalciferol, Vitamin E, DL-α-Tocopherol, Tocopherol-E-Acetat, Tocopherolhydrogensuccinat, Vitamin K₁, Esculin, Thiamin, Nicotinsäure, Pyridoxin, Pyridoxal, Pyridoxamin, Panthothensäure, Biotin, Folsäure und Cobalamin enthält.

## Claims

1. Cosmetic formulation comprising
a) one or more compounds chosen from compounds of the formulae Ia and Ib 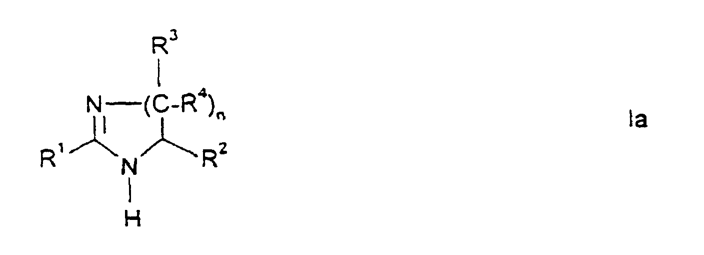 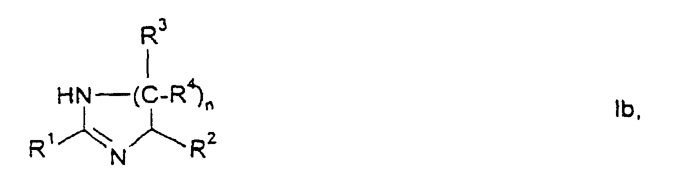 the physiologically compatible salts of the compounds of the formulae Ia and Ib, and the stereoisomeric forms of the compounds of the formulae Ia and Ib, where
R¹ is H or alkyl,
R² is H, COOH, COO-alkyl or CO-NH-R⁵,
R³ and R⁴ are in each case independently of one another H or OH,
n is 1, 2 or 3,
alkyl is an alkyl radical having 1 to 4 carbon atoms, and
R⁵ is H, alkyl, an amino acid radical, dipeptide radical or tripeptide radical,
b) one or more enzymes
and
c) auxiliaries and carriers
**characterized in that** it additionally comprises one or more vitamins and/or vitamin derivatives chosen from vitamin A, vitamin A propionate, vitamin A palmitate, vitamin A acetate, retinol, vitamin B, thiamine chloride hydrochloride, riboflavin, nicotinamide, vitamin C, vitamin D, ergocalciferol, vitamin E, DL-α-tocopherol, tocopherol E acetate, tocopherol hydrogensuccinate, vitamin K₁, esculin, thiamine, nicotinic acid, pyridoxine, pyridoxal, pyridoxamine, panthothenic acid, biotin, folic acid and cobalamin.

2. Cosmetic formulation according to Claim 1, **characterized in that** the compounds of the formulae Ia and Ib are chosen from the compounds (S)-1,4,5,6-tetrahydro-2-methyl-4-pyrimidine carboxylic acid and (S,S)-1,4,5,6-tetrahydro-5-hydroxy-2-methyl-4-pyrimidine carboxylic acid.

3. Cosmetic formulation according to one of Claims 1 or 2, **characterized in that** the enzymes are chosen from superoxide dismutase, protease, lipase, pepsin, trypsin, chymotrypsin, elastase, diastase, catalase, dehydrogenase, urease, lysozyme, neuraminidase, peroxidase, glutathione peroxidase, phosphatase, dismutase, papain, bromelain, alcalase, aminopeptidase K, aminopeptidase M, carboxypeptidase A, carboxypeptidase B, carboxypeptidase Y, clostripain, collagenase, ficine, leucine amidepeptidase, lytic enzyme, pronase E and proteinase K.

4. Cosmetic formulation according to one of Claims 1 to 3, **characterized in that** the proportion of the compounds chosen from the compounds of the formulae Ia and Ib, the physiologically compatible salts of the compounds of the formulae Ia and Ib, and the stereoisomeric forms of the compounds of the formulae Ia and Ib is from 0.0001 to 50% by weight, based on the total cosmetic formulation, and the proportion of the enzymes is from 0.0001 to 10% by weight, based on the total cosmetic formulation.

5. Cosmetic formulation according to one of Claims 1 to 4, **characterized in that** the proportion of the compounds chosen from the compounds of the formulae Ia and Ib, the physiologically compatible salts of the compounds of the formulae Ia and Ib, and the stereoisomeric forms of the compounds of the formulae Ia and Ib is from 0.0001 to 50% by weight, based on the total cosmetic formulation, and the proportion of the vitamins and/or vitamin derivatives is from 0.001 to 20% by weight, based on the total cosmetic formulation.

6. Cosmetic formulation according to one of Claims 1 to 5, **characterized in that** it is in the form of a solution, a suspension, an emulsion, a paste, an ointment, a gel, a cream, a lotion, a powder, a soap, a surfactant-containing cleansing preparation, an oil, a lipstick, a lipcare stick, a mascara, an eyeliner, eyeshadows, blusher, a powder, emulsion or wax foundation, a sunscreen, presun and aftersun preparation or a spray.

7. Use of
a) one or more compounds chosen from compounds of the formulae Ia and Ib 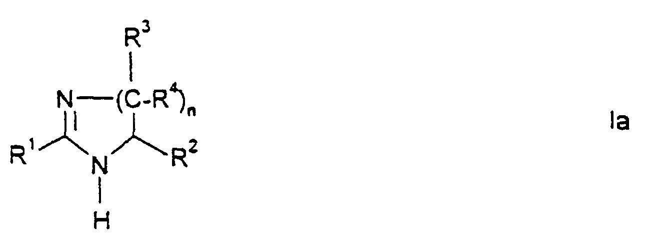 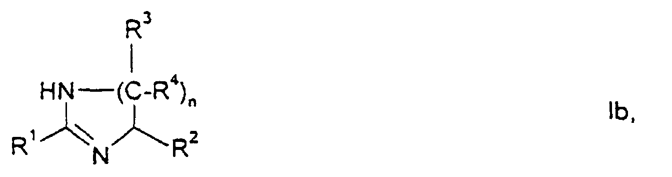 the physiologically compatible salts of the compounds of the formulae Ia and Ib, and the stereoisomeric forms of the compounds of the formulae Ia and Ib, where
R¹ is H or alkyl,
R² is H, COOH, COO-alkyl or CO-NH-R⁵,
R³ and R⁴ are in each case independently of one another H or OH,
n is 1, 2 or 3,
alkyl is an alkyl radical having 1 to 4 carbon atoms, and
R⁵ is H, alkyl, an amino acid radical, dipeptide radical or tripeptide radical
for the protection and/or stabilization of one or more substances present in a cosmetic formulation, chosen from vitamins and vitamin derivatives.

8. Use of
a) one or more compounds chosen from compounds of the formulae Ia and Ib 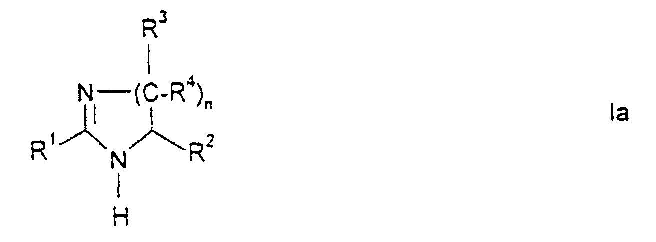 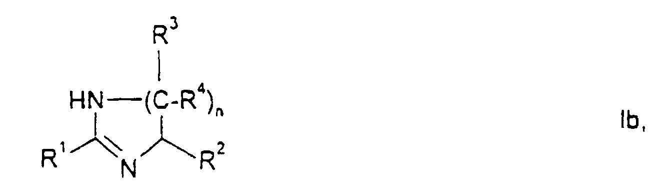 the physiologically compatible salts of the compounds of the formulae Ia and Ib, and the stereoisomeric forms of the compounds of the formulae Ia and Ib, where
R¹ is H or alkyl,
R² is H, COOH, COO-alkyl or CO-NH-R⁵,
R³ and R⁴ are in each case independently of one another H or OH,
n is 1, 2 or 3,
alkyl is an alkyl radical having 1 to 4 carbon atoms, and
R⁵ is H, alkyl, an amino acid radical, dipeptide radical or tripeptide radical,
b) one or more enzymes and
c) auxiliaries and carriers
for the preparation of a cosmetic formulation, **characterized in that** the formulation additionally comprises one or more vitamins and/or vitamin derivatives chosen from vitamin A, vitamin A propionate, vitamin A palmitate, vitamin A acetate, retinol, vitamin B, thiamine chloride hydrochloride, riboflavin, nicotinamide, vitamin C, vitamin D, ergocalciferol, vitamin E, DL-α-tocopherol, tocopherol E acetate, tocopherol hydrogensuccinate, vitamin K₁, esculin, thiamine, nicotinic acid, pyridoxine, pyridoxal, pyridoxamine, panthothenic acid, biotin, folic acid and cobalamin.

9. Use according to Claim 8, **characterized in that** the cosmetic formulation is in the form of a solution, a suspension, an emulsion, a paste, an ointment, a gel, a cream, a lotion, a powder, a soap, a surfactant-containing cleansing preparation, an oil, a lipstick, a lipcare stick, a mascara, an eyeliner, eyeshadows, blusher, a powder, emulsion or wax foundation, a sunscreen, presun and aftersun preparation or a spray.

10. Use according to one of Claims 7 to 9, **characterized in that** the compounds of the formulae Ia and Ib are chosen from the compounds (S)-1,4,5,6-tetrahydro-2-methyl-4-pyrimidinecarboxylic acid and (S,S)-1,4,5,6-tetrahydro-5-hydroxy-2-methyl-4-pyrimidinecarboxylic acid.

11. Use according to one of Claims 7 to 10, **characterized in that** that the enzymes are chosen from superoxide dismutase, protease, lipase, pepsin, trypsin, chymotrypsin, elastase, diastase, catalase, dehydrogenase, urease, lysozyme, neuraminidase, peroxidase, glutathione peroxidase, phosphatase, dismutase, papain, bromelain, alcalase, aminopeptidase K, aminopeptidase M, carboxypeptidase A, carboxypeptidase B, carboxypeptidase Y, clostripain, collagenase, ficine, leucine amidepeptidase, lytic enzyme, pronase E and proteinase K.

12. Use according to one of Claims 7, 10 or 11, **characterized in that** the vitamins and/or vitamin derivatives are chosen from vitamin A, vitamin A propionate, vitamin A palmitate, vitamin A acetate, retinol, vitamin B, thiamine chloride hydrochloride, riboflavin, nicotinamide, vitamin C, vitamin D, ergocalciferol, vitamin E, DL-α-tocopherol, tocopherol E acetate, tocopherol hydrogensuccinate, vitamin K₁, esculin, thiamine, nicotinic acid, pyridoxine, pyridoxal, pyridoxamine, panthothenic acid, biotin, folic acid and cobalamin.

13. Method of preparing a cosmetic formulation, **characterized in that**
a) one or more compounds chosen from compounds of the formulae Ia and Ib 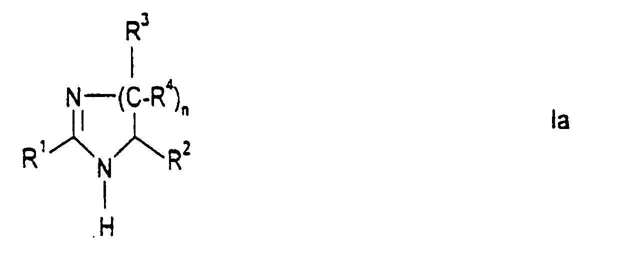 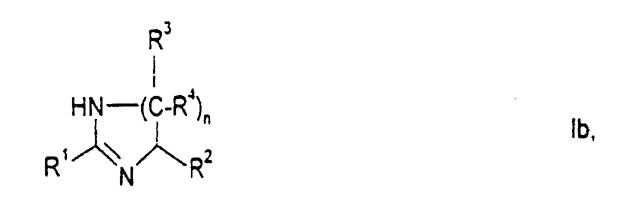 the physiologically compatible salts of the compounds of the formulae Ia and Ib, and the stereoisomeric forms of the compounds of the formulae Ia and Ib, where
R¹ is H or alkyl,
R² is H, COOH, COO-alkyl or CO-NH-R⁵,
R³ and R⁴ are in each case independently of one another H or OH,
n is 1, 2 or 3,
alkyl is an alkyl radical having 1 to 4 carbon atoms, and
R⁵ is H, alkyl, an amino acid radical, dipeptide radical or tripeptide radical
and
b) one or more enzymes
with auxiliaries and/or carriers are converted to a suitable cosmetic formulation form, which additionally comprises one or more vitamins and/or vitamin derivatives chosen from vitamin A, vitamin A propionate, vitamin A palmitate, vitamin A acetate, retinol, vitamin B, thiamine chloride hydrochloride, riboflavin, nicotinamide, vitamin C, vitamin D, ergocalciferol, vitamin E, DL-α-tocopherol, tocopherol E acetate, tocopherol hydrogensuccinate, vitamin K₁, esculin, thiamine, nicotinic acid, pyridoxine, pyridoxal, pyridoxamine, panthothenic acid, biotin, folic acid and cobalamin.

## Revendications

1. Formulation cosmétique contenant
a) un ou plusieurs composés, sélectionnés parmi les composés répondant aux formules Ia et Ib 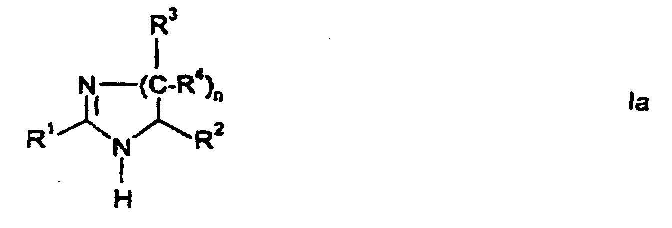 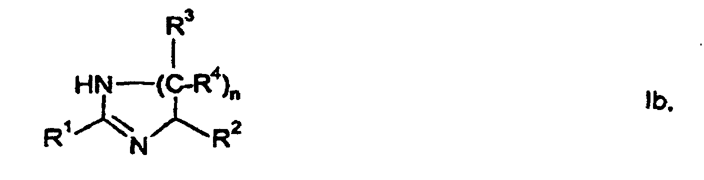 ainsi que parmi les sels, compatibles du point de vue physiologique, des composés répondant aux formules Ia et Ib et les formes stéréoisomères des composés répondant aux formules Ia et Ib, formules dans lesquelles :
R¹ représente H ou un groupe alkyle ;
R² représente H, COOH, un groupe COO-alkyle, ou CO-NH-R⁵ ;
R³ et R⁴ représentent chacun, indépendamment H ou OH ;
n vaut 1, 2 ou 3 ;
"alkyle" représente un radical alkyle comportant de 1 à 4 atomes de carbone ;
R⁵ représente H, un groupe alkyle, un radical aminoacide, un radical dipeptide ou un radical tripeptide ;
b) une ou plusieurs enzymes ;
c) des adjuvants et substances de support ;
**caractérisée en ce qu'**elle contient en outre une ou plusieurs vitamines et/ou dérivés de vitamines sélectionnées dans le groupe contenant : vitamine A, propionate de vitamine A, palmitate de vitamine A, acétate de vitamine A, rétinol, vitamine B, chlorhydrate de chlorure de thiamine, riboflavine, nicotinamide, vitamine C, vitamine D, ergocalciférol, vitamine E, DL-α-tocophérol, acétate de tocophérol E, succinate de tocophérol hydrogène, vitamine K₁, esculine, thiamine, acide nicotique, pyridoxine, pyridoxal, pyridoxamine, acide pantothénique, biotine, acide folique et cobalamine.

2. Formulation cosmétique selon la revendication 1,
**caractérisée en ce que** les composés répondant aux formules Ia et Ib sont sélectionnés parmi l'acide carbonique de (S)-1,4,5,6-tétrahydro-2-méthyl-4-pyrimidine et l'acide carbonique de (S,S)-1,4,5,6-tétrahydro-5-hydroxy-2-méthyl-4-pyrimidine.

3. Formulation cosmétique selon l'une des revendications 1 ou 2, **caractérisée en ce que** les enzymes sont sélectionnées parmi dismutase de peroxyde, protéase, lipase, pepsine, trypsine, chymotrypsine, élastase, diastase, catalase, déshydrogénase, uréase, lysozyme, neuraminidase, péroxydase, glutathionperoxydase, phosphatase, dismutase, papaïne, broméline, alcalase, aminopeptidase K, aminopeptidase M, carboxypeptidase A, carboxypeptidase B, carboxypeptidase Y, clostripaïne, collagénase, ficine, leucine-amidopeptidase, enzyme lytique, pronase E et protéinase K.

4. Formulation cosmétique selon l'une des revendications 1 à 3, **caractérisée en ce que** la proportion des composés, sélectionnés parmi les composés répondant aux formules Ia et Ib, les sels compatibles du point de vue physiologique des composés répondant aux formules Ia et Ib et les formes stéréoisomères des composés répondant aux formules Ia et Ib, est de 0,0001 à 50 % en poids par rapport à la formulation cosmétique totale, et la proportion des enzymes est de 0,0001 à 10 % en poids par rapport à la formulation cosmétique totale.

5. Formulation cosmétique selon l'une des revendications 1 à 4, **caractérisée en ce que** la proportion des composés, sélectionnés parmi les composés répondant aux formules Ia et Ib, les sels compatibles du point de vue physiologique des composés répondant aux formules Ia et Ib et les formes stéréoisomères des composés répondant aux formules Ia et Ib, est de 0,0001 à 50 % en poids par rapport à la formulation cosmétique totale, et la proportion des vitamines et/ou des dérivés de vitamines est de 0,001 à 20 % en poids par rapport à la formulation cosmétique totale.

6. Formulation cosmétique selon l'une des revendications 1 à 5, **caractérisée en ce qu'**elle se présente sous la forme d'une solution, d'une suspension, d'une émulsion, d'une pâte, d'une pommade, d'un gel, d'une crème, d'une lotion, d'une poudre, d'un savon, d'un produit de nettoyage contenant un agent tensioactif, d'une huile, d'un bâton de rouge à lèvres, d'un bâton de baume à lèvres, d'un mascara, d'un eyeliner, d'un fard à paupières, d'un rouge, d'un maquillage à base de poudre, d'émulsion ou de cire, d'un produit de protection solaire, de produits solaires et après soleil, ou d'un spray.

7. Utilisation
a) d'un ou plusieurs composés, sélectionnés parmi les composés répondant aux formules Ia et Ib 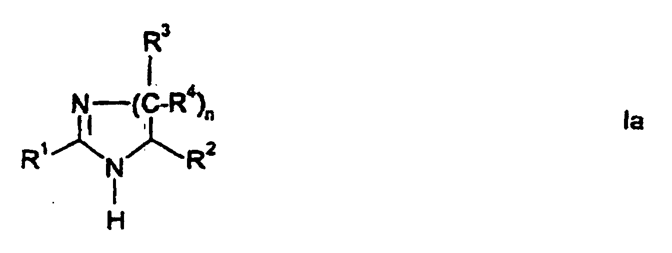 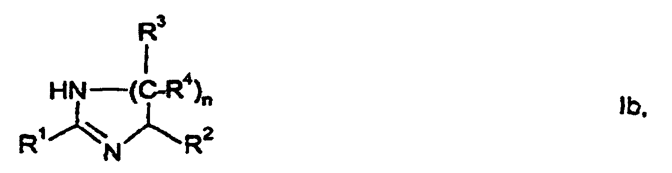 ainsi que parmi les sels, compatibles du point de vue physiologique, des composés répondant aux formules Ia et Ib et les formes stéréoisomères des composés répondant aux formules Ia et Ib, formules dans lesquelles :
R¹ représente H ou un groupe alkyle ;
R² représente H, COOH, un groupe COO-alkyle, ou CO-NH-R⁵ ;
R³ et R⁴ représentent chacun, indépendamment H ou OH ;
n vaut 1, 2 ou 3 ;
"alkyle" représente un radical alkyle comportant de 1 à 4 atomes de carbone ;
R⁵ représente H, un groupe alkyle, un radical aminoacide, un radical dipeptide ou un radical tripeptide,
pour la protection et/ou la stabilisation d'une ou plusieurs substances contenues dans une formulation cosmétique et sélectionnées parmi les vitamines et dérivés de vitamines.

8. Utilisation
a) d'un ou plusieurs composés sélectionnés parmi les composés répondant aux formules Ia et Ib 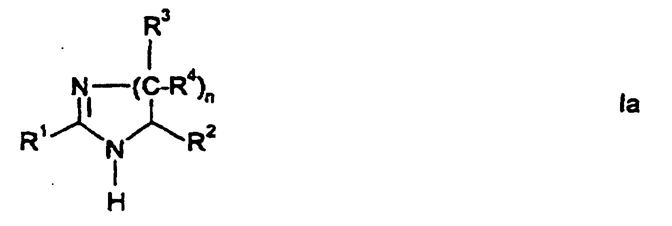 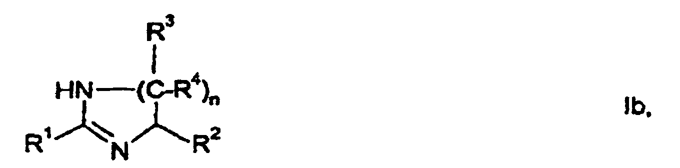 ainsi que parmi les sels, compatibles du point de vue physiologique, des composés répondant aux formules Ia et Ib et les formes stéréoisomères des composés répondant aux formules Ia et Ib, formules dans lesquelles :
R¹ représente H ou un groupe alkyle ;
R² représente H, COOH, un groupe COO-alkyle, ou CO-NH-R⁵ ;
R³ et R⁴ représentent chacun, indépendamment H ou OH ;
n vaut 1, 2 ou 3 ;
"alkyle" représente un radical alkyle comportant de 1 à 4 atomes de carbone ;
R⁵ représente H, un groupe alkyle, un radical aminoacide, un radical dipeptide ou un radical tyripeptide ;
b) d'une ou plusieurs enzymes ;
c) d'adjuvants et substances de support
pour la préparation d'une formulation cosmétique **caractérisée en ce qu'**elle contient en outre une ou plusieurs vitamines et/ou dérivés de vitamines sélectionnées parmi vitamine A, propionate de vitamine A, palmitate de vitamine A, acétate de vitamine A, rétinol, vitamine B, chlorhydrate de chlorure de thiamine, riboflavine, nicotinamide, vitamine C, vitamine D, ergocalciférol, vitamine E, DL-α-tocophérol, acétate de tocophérol E, succinate de tocophérol hydrogène, vitamine K₁, esculine, thiamine, acide nicotique, pyridoxine, pyridoxal, pyridoxamine, acide pantothénique, biotine, acide folique et cobalamine.

9. Utilisation selon la revendication 8, **caractérisée en ce que** la formulation cosmétique se présente sous la forme d'une solution, d'une suspension, d'une émulsion, d'une pâte, d'une pommade, d'un gel, d'une crème, d'une lotion, d'une poudre, d'un savon, d'un produit de nettoyage contenant un agent tensioactif, d'une huile, d'un bâton de rouge à lèvres, d'un bâton de baume à lèvres, d'un mascara, d'un eyeliner, d'un fard à paupières, d'un rouge, d'un maquillage à base de poudre, d'émulsion ou de cire, d'un produit de protection solaire, de produits solaires et après soleil, ou d'un spray.

10. Utilisation selon l'une des revendications 7 à 10, **caractérisée en ce que** les composés répondant aux formules Ia et Ib sont sélectionnés parmi l'acide carbonique de (S)-1,4,5,6-tétrahdro-2-méthyl-4-pyrimidine et l'acide carbonique de (S,S)-1,4,5,6-tétrahydro-5-hydroxy-2-méthyl-4-pyrimidine.

11. Utilisation selon l'une des revendications 7 à 10, **caractérisée en ce que** les enzymes sont sélectionnés parmi superoxyde dismutase, protéase, lipase, pepsine, trypsine, chymotrypsine, élastase, diastase, catalase, déshydrogénase, uréase, lysozyme, neuraminidase, péroxydase, glutathionperoxydase, phosphatase, dismutase, papaïne, broméline, alcalase, aminopeptidase K, aminopeptidase M, cabroxypeptidase A, carboxypeptidase B, carboxypeptidase Y, clostripaïne, collagénase, ficine, leucine-amidopeptidase, enzyme lytique, pronase E et protéinase K.

12. Utilisation selon l'une des revendications 7, 10 ou 11, **caractérisée en ce que** les vitamines et/ou dérivés de vitamines sont sélectionnées parmi vitamine A, propionate de vitamine A, palmitate de vitamine A, acétate de vitamine A, rétinol, vitamine B, chlorhydrate de chlorure de thiamine, riboflavine, nicotinamide, vitamine C, vitamine D, ergocalciférol, vitamine E, DL-a-tocophérol, acétate de tocophérol E, succinate de tocophérol hydrogène, vitamine K₁, esculine, thiamine, acide nicotique, pyridoxine, pyridoxal, pyridoxamine, acide pantothénique, biotine, acide folique et cobalamine.

13. Procédé de préparation d'une formulation cosmétique, **caractérisé en ce que** l'on transforme :
a) un ou plusieurs composés, sélectionnés parmi les composés répondant aux formules Ia et Ib : 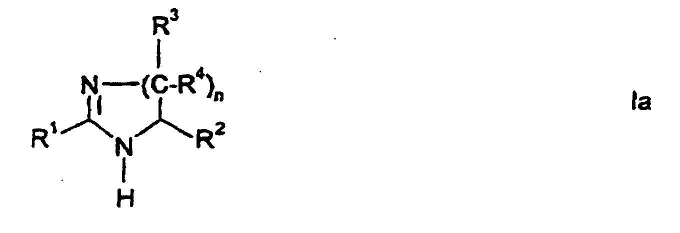 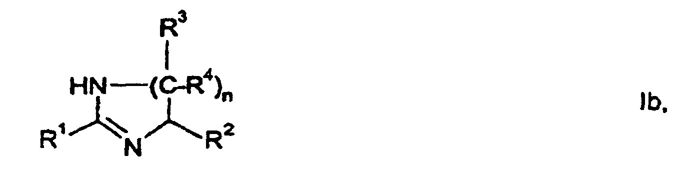 ainsi que parmi les sels, compatibles du point de vue physiologique, des composés répondant aux formules Ia et Ib et les formes stéréoisomères des composés répondant aux formules Ia et Ib, formules dans lesquelles
R¹ représente H ou un groupe alkyle ;
R² représente H, COOH, un groupe COO-alkyle, ou CO-NH-R⁵ ;
R³ et R⁴ représentent, indépendamment H ou OH ;
n vaut 1, 2 ou 3 ;
"alkyle" représente un radical alkyle comportant de 1 à 4 atomes de carbone ;
R⁵ représente H, un groupe alkyle, un radical aminoacide, un radical dipeptide ou un radical tyripeptide ; et
b) une ou plusieurs enzymes,
avec des adjuvants et substances de support, en une formulation cosmétique appropriée qui contient en outre une ou plusieurs vitamines et/ou dérivés de vitamines sélectionnées parmi vitamine A, propionate de vitamine A, palmitate de vitamine A, acétate de vitamine A, rétinol, vitamine B, chlorhydrate de chlorure de thiamine, riboflavine, nicotinamide, vitamine C, vitamine D, ergocalciférol, vitamine E, DL-α-tocophérol, acétate de tocophérol E, succinate de tocophérol hydrogène, vitamine K₁, esculine, thiamine, acide nicotique, pyridoxine, pyridoxal, pyridoxamine, acide pantothénique, biotine, acide folique et cobalamine.
